# EUROPÄISCHE PATENTANMELDUNG

(11) **EP 1 092 713 A2**
(43) Veröffentlichungstag der Anmeldung: **18.04.2001**
(21) Anmeldenummer: 00127106.3
(22) Anmeldetag: 19.03.1996
(51) Int. Cl.: C07D 295/12, C08G 18/20

(54) **Piperidinopentanamine und ihre Verwendung als Katalysator bei der Herstellung von Urethanen**

(30) Priorität: 26.04.1995 CH 119095
(62) Teilanmeldung aus: 96909998.5
(71) Anmelder: LONZA AG, CH-3945 Gampel/Wallis (CH)
(72) Erfinder: Heveling, Josef, 3904 Naters (CH); Gerhard, Andreas, 3930 Visp (CH)
(74) Vertreter: Winter, Brandl, Fürniss, Hübner, Röss, Kaiser, Polte Partnerschaft

(57) **Zusammenfassung**

Methyl-5-(3-methylpiperidino)pentanamine der allgemeinen Formel 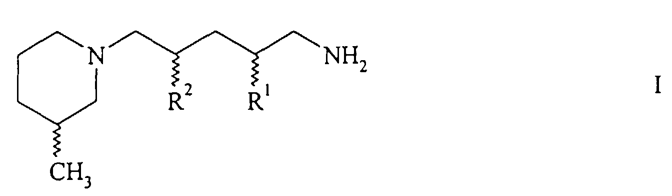 worin einer der beiden Reste R¹, R² Wasserstoff und der andere Methyl bedeutet, werden aus 2-Methylglutaronitril durch zweistufige Hydrierung hergestellt. Die Verbindungen eignen sich als Katalysatoren für die Herstellung von Polyurethanen.

## Beschreibung

Die vorliegende Erfindung betrifft neue Piperidinopentanamine, sowohl einzeln als auch als Gemisch, namentlich das 2-Methyl-5-(3-methylpiperidino)-pentanamin und das isomere 4-Methyl-5-(3-methylpiperidino)-pentanamin, die sich durch die gemeinsame Formel 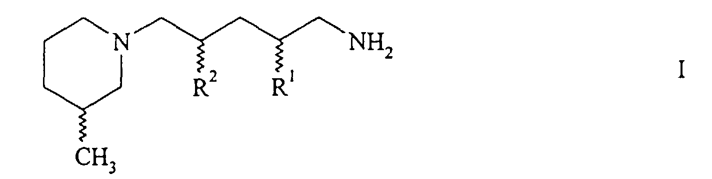 darstellen lassen. Hierin steht jeweils einer der beiden Reste R¹ und R² für Wasserstoff und der andere für Methyl. Im Fall des 2-Methyl-5-(3-methylpiperidino)-pentanamins (Ia) ist R¹ = Methyl und R² = H, im Fall des 4-Methyl-5-(3-methylpiperidino)pentanamins (Ib) ist R¹ = H und R² = Methyl. Beide Verbindungen können jeweils in vier verschiedenen stereoisomeren Formen (zwei Diastereomerenpaare) vorliegen. Hier und im folgenden stehen die Formeln und die zugehörigen Verbindungsnamen jeweils für alle möglichen Stereoisomeren.

Die Erfindung betrifft weiterhin die Verwendung der Verbindungen als Katalysatoren für die Herstellung von Polyurethanen aus Polyisocyanaten und Polyolen.

Für die Herstellung von Polyurethanen werden üblicherweise Stickstoffbasen, insbesondere tertiäre Amine wie beispielsweise 1,4-Diazabicyclo[2.2.2]octan (DABCO^{®}), cyclische Amidine wie beispielsweise 1,8-Diazabicyclo[5.4.0]undec-7-en ("DBU") oder auch Guanidinderivate wie Tetramethylguanidin als Katalysatoren eingesetzt. Für die Eignung als Katalysator sind u.a. die Basizität, Löslichkeitsverhalten und Flüchtigkeit massgebend. Während die Basizität in erster Linie die katalytische Wirksamkeit bestimmt, sind Löslichkeitsverhalten und Flüchtigkeit für die Verarbeitungseigenschaften ebenfalls von Bedeutung. Zu hohe Flüchtigkeit beispielsweise kann durch Freisetzung in die Atmosphäre bei der Herstellung der Polyurethane oder ihrem Gebrauch zur Belästigung oder Gefährdung von Personen führen.

Aufgabe der vorliegenden Erfindung war es, neue Amine bereitzustellen, die als Katalysator zur Herstellung von Polyurethanen geeignet sind, günstige Gebrauchseigenschaften aufweisen und einfach und kostengünstig herzustellen sind.

Erfindungsgemäss wird die Aufgabe durch die Piperidinopentanamine nach Anspruch 1 und das Herstellungsverfahren nach Anspruch 4 gelöst.

Es wurde gefunden, dass durch Umsetzung des bei der Adiponitrilherstellung als Nebenprodukt anfallenden 2-Methylglutaronitrils mit Wasserstoff in Gegenwart eines Katalysators in einer Stufe neben anderen Produkten ein Gemisch der beiden isomeren Methyl-5-(3-methyl-piperidino)pentannitrile der allgemeinen Formel 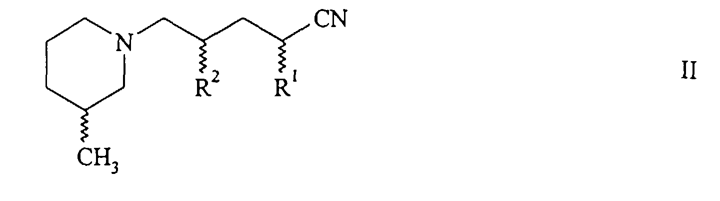 worin einer der beiden Reste R¹, R² Wasserstoff und der andere Methyl bedeutet, hergestellt und in einer zweiten Stufe in Gegenwart eines zweiten Katalysators zu dem Gemisch der beiden isomeren Methyl-5-(3-methylpiperidino)pentanamine Ia und Ib hydriert werden kann.

Das Isomerengemisch Ia/Ib kann gegebenenfalls destillativ oder chromatographisch in die beiden Strukturisomeren Ia und Ib aufgetrennt werden, wird aber vorzugsweise als solches verwendet.

Als Katalysator für die erste Stufe wird vorzugsweise ein Palladium-Trägerkatalysator eingesetzt, besonders bevorzugt ist Palladium auf Aluminiumoxid.

Die Umsetzung des 2-Methylglutaronitrils mit Wasserstoff wird vorzugsweise bei einer Temperatur von 100 bis 250 °C und einem Druck von 20 bis 70 bar durchgeführt.

Es hat sich als vorteilhaft erwiesen, dem 2-Methylglutaronitril etwas 3-Methylpiperidin zuzusetzen. Vorzugsweise wird das 3-Methylpiperidin in einer Menge von 1 bis 5 mol auf 1 mol 2-Methylglutaronitril zugegeben. Die Selektivität der Reaktion wird hierdurch wesentlich gesteigert. 3-Methylpiperidin kann nach bekannten Verfahren ebenfalls aus 2-Methylglutaro-nitril hergestellt werden (WO 90/00 546).

Die Umsetzung des 2-Methylglutaronitrils mit Wasserstoff wird vorzugsweise kontinuierlich in einem Festbettreaktor durchgeführt.

Die Hydrierung der Methyl-5-(3-methylpiperidino)pentannitrile II zu den Methyl-5-(3-methylpiperidino)pentanaminen Ia/Ib wird vorteilhaft in Gegenwart von Ammoniak durchgeführt, um unerwünschte Nebenreaktionen zu unterdrücken.

Als Katalysatoren für diese zweite Stufe können grundsätzlich alle Katalysatoren eingesetzt werden, die sich für die Hydrierung von Nitrilen zu Aminen eignen, also beispielsweise Palladium, Platinum, Rhodium, Cobalt, Nickel oder Nickel-Borid.

Vorzugsweise wird als Katalysator für die zweite Stufe ein Rhodium-Trägerkatalysator eingesetzt, besonders bevorzugt ist Rhodium auf Aktivkohle.

Die Hydrierung der Methyl-5-(3-methylpiperidino)pentannitrile II an einem Rhodium-Trägerkatalysator wird vorzugsweise bei einer Temperatur von 5 bis 100 °C und einem Druck von 1 bis 50 bar durchgeführt.

Als Lösungsmittel für die zweite Stufe können die für die Hydrierung von Nitrilen zu Aminen üblichen Lösungsmittel eingesetzt werden. Vorzugsweise werden Alkohole wie beispielsweise Ethanol oder *tert*-Butylalkohol eingesetzt.

Die erfindungsgemässen Methyl-5-(3-methylpiperidino)pentanamine Ia/Ib können, wie bereits eingangs erwähnt, als Katalysatoren zur Herstellung von Polyurethanen eingesetzt werden.

Die nachfolgenden Beispiele verdeutlichen die Herstellung und Verwendung der erfindungsgemässen Verbindungen.

### Beispiele:

### Beispiel 1

In einen Reaktor (13 mm Ø) wurden 3 g Pd/A1₂O₃-Katalysator (1% Pd, Korngrösse 0,315-1,0 mm) eingefüllt. Der Reaktor wurde im Wasserstoffstrom (120 ml/min, bezogen auf Normaldruck) bei 50 bar auf die Reaktionstemperatur von 150 °C aufgeheizt. Dann wurde dem Wasserstoff eine Mischung aus 3-Methylpiperidin und 2-Methylglutaronitril im molaren Verhältnis von 2:1 zudosiert. Der Durchsatz betrug 2,1 g Edukt pro g Katalysator und Stunde. Das Produktgemisch nach Verlassen des Reaktors und Abtrennen des Wasserstoffs enthielt gemäss GC 44,8% 2(4)-Methyl-5-(3-methylpiperidino)pentannitril (Isomerengemisch), 30,2% 3-Methylpiperidin und 24,4% 1,5-Bis(3-methylpiperidino)-2-methylpentan sowie 0,6% nicht identifizierte Produkte.
Das Gemisch wurde über 260 h Reaktionsdauer gesammelt und anschliessend im Vakuum fraktioniert. Bei 87 °C/2,5 mbar ging das Methyl-5-(3-methylpiperidino)pentannitril in einer Reinheit von 99,5% (GC) über. Gemäss NMR-Analyse handelte es sich um ein Gemisch aus 85% 2-Methyl- und 15% 4-Methyl-Verbindung.

| Analytische Daten: | | | | | | | |
|---|---|---|---|---|---|---|---|
| C₁₂H₂₂N₂ | Berechnet | C | 74,2 | H | 11,4 | N | 14,4 |
| | Gefunden | C | 74,2 | H | 11,6 | N | 14,9 |

- ¹H NMR (CDCl₃, 400 MHz) δ: (Hauptkomponente): 1,40-2,85 (m, 16H, CH + CH₂); 1,32 (d, 3 H, C*H*₃-CH-CN); 0,86 (d, 3 H, Ring-CH₃).
- ¹³C NMR (CDCl₃, 100 MHz) δ: (Hauptkomponente): 123,01 (s); 62,20 (t); 58,28 (t); 54,12 (t); 33,13 (t); 32,23 (t); 31,19 (d); 25,62 (t);

### Beispiel 2

Es wurde verfahren wie in Beispiel 1 beschrieben, jedoch betrug die Reaktionstemperatur nur 125 °C.
Das Produktgemisch enthielt ca. 50% 2-Methyl-5-(3-methylpiperidino)pentannitril, 8% 4-Methyl-5-(3-methylpiperidino)pentannitril, 32% 3-Methylpiperidin, 6% 1,5-Bis(3-methyl-piperidino)-2-methylpentan, 1% 2-Methylglutaronitril und 3% nicht identifizierte Produkte.

Das Gemisch wurde über 284 h Reaktionsdauer gesammelt und anschliessend im Vakuum fraktioniert. Bei 87 °C/2,5 mbar ging das Methyl-5-(3-methylpiperidino)pentannitril in einer Reinheit von 99,3% (GC) über. Gemäss NMR-Analyse handelte es sich um ein Gemisch aus 86% der 2-Methyl- und 14% der 4-Methyl-Verbindung.

### Beispiel 3

In einen Reaktor (13 mm Ø) wurden 3 g eines Pd/MgCl₂/Al₂O₃-Katalysators (1% Pd, 1,2% Mg, Korngrösse 0,315-1,0 mm) eingefüllt. Der Reaktor wurde im Wasserstoffstrom (120 ml/min, bezogen auf Normaldruck) bei 50 bar auf die Reaktionstemperatur von 160 °C aufgeheizt. Dann wurde dem Wasserstoff 99,8%iges 2-Methylglutaronitril zudosiert. Der Durchsatz betrug 2,1 g Edukt pro g Katalysator und Stunde. Gemäss GC enthielt der Produktstrom 25,8% 2(4)-Methyl-5-(3-methylpiperidino)pentannitril (Isomerengemisch), 52,1% 1,5-Bis(3-methylpiperidino)-2-methylpentan, 3,7% 3-Methylpiperidin und 18,4% nicht identifizierte Produkte.
Die Selektivität der Reaktion zu den gewünschten Nitrilen war im Vergleich zu den Beispielen 1 und 2 (mit Zusatz von 3-Methylpiperidin) deutlich geringer.

### Beispiel 4

In 100 g trockenen *tert*-Butylalkohol wurde unter Kühlung Ammoniak eingeleitet, bis eine Konzentration von 13,7% NH₃ erreicht war. Ein 100 ml Autoklav mit magnetgetriebenem Rührer wurde mit Stickstoff gespült und vorgekühlt. Dann wurden 30 g der Lösung von Ammoniak in *tert*-Butylalkohol, 4 g Rhodium/Aktivkohle (5% Rh) und 30 g des Gemischs der Methyl-5-(3-methylpiperidino)pentannitrile aus Beispiel 1 eingefüllt. Der Autoklav wurde geschlossen und unter Rühren erwärmt. Bei Raumtemperatur wurden 10 bar Wasserstoff aufgepresst, dann wurde die Temperatur weiter auf 50 °C erhöht. Nach 6 h war gemäss GC ein Umsatz von 99,2% und eine Ausbeute von 87,3% erreicht.

Das Reaktionsgemisch wurde im Vakuum destilliert, wobei das Isomerengemisch 2(4)-Methyl-5-(3-methylpiperidino)pentanamin bei 104 °C/3 mbar in einer Reinheit von 99,5% erhalten wurde. Das Gemisch enthielt gemäss GC die 2-Methyl- und die 4-Methyl-Verbindung im Verhältnis 2,6:1.

| Analytische Daten: | | | | | | | |
|---|---|---|---|---|---|---|---|
| C₁₂H₂₆N₂ | Berechnet | C | 72,7 | H | 13,2 | N | 14,1 |
| | Gefunden | C | 72,9 | H | 13,1 | N | 14,6 |

- ¹H NMR (CDCl₃, 400 MHz) δ=: 0,6-0,8 (CH₃, 1 Methylen-H);
1,0-1,25 (NH₂, 1 Methylen-H);
1,30-1,85;
2,05; 2,27;
2,55;
2,65-2,80.
Die NMR-Spektren sind wegen des Vorliegens mehrerer diastereomerer Formen sehr komplex.

### Beispiele 5-8

Das Isomerengemisch 2-Methyl-5-(3 -methylpiperidino)pentanamin/4-Methyl-5-(3-methyl-piperidino)pentanamin (2,6:1) aus Beispiel 4 wurde als Polyurethan-Katalysator eingesetzt:

### Abkürzungen:

- VL:: Desmodur^{®} VL von Bayer, aromatisches Diisocyanat mit ca. 32% -NCO
- D550U:: Desmophen^{®} 550U, Polypropylenglykol von Bayer, trifunktionell mit 10,5%-OH
- DBU:: Diazabicyclo[5.4.0]undec-7-en
- MMPPA:: Gemisch aus 2-Methyl- und 4-Methyl-5-(3-methylpiperidino)pentanamin (2,6:1)

Als Vergleichskatalysator diente DBU. Desmophen^{®} wurde mit dem Amin (DBU oder MMPPA) vorgelegt und gut gemischt. Es entstand eine Lösung. In Beispiel 7 und 8 wurde zusätzlich Wasser zugegeben. Es entstand eine Emulsion/Lösung. Eine eingewogene Menge Isocyanat (VL) wurde zum Zeitpunkt *t* = 0 unter heftigem Rühren zugesetzt. Es wurde der Zeitpunkt notiert,
- zu dem die Lösung nicht mehr trübe war,
- zu dem eine deutliche Erwärmung feststellbar war,
- zu dem die Mischung fest wurde.

Die Resultate (MMPPA im Vergleich zu DBU) gehen aus der folgenden Tabelle 1 hervor:

**Tabelle 1:**

| Bsp. Nr. | *t* [s] nicht mehr trübe | *t* [s] Erwärmung | *t* [s] fest | VL [g] | D550U [g] | DBU [g] | MMPPA [g] | H₂O [g] | Vol. [ml] | Bemerkungen (Farbe etc.) |
|---|---|---|---|---|---|---|---|---|---|---|
| 5 | 550 | 360 | 820 | 50,0 | 55,0 | 0,1 | - | - | | braun/ocker, hart |
| 6 | 295 | 145 | 550 | 50,0 | 55,0 | - | 0,1 | - | | ocker, hart |
| 7 | 200 | 180 | 1180 | 50,0 | 45,0 | 0,1 | - | 0,5 | 400 | ocker, Hartschaum |
| 8 | 175 | 150 | 960 | 50,0 | 45,0 | - | 0,1 | 0,5 | 300 | ocker, hart |

### Beispiele 9-12

Das Polymer aus den Beispielen 5 und 6 wurde zu feinen Flocken zerhobelt und jeweils 4 g davon in 45 g Ethanol aufgeschlämmt und täglich bewegt. Mit GC wurde bestimmt, wieviel des Katalysators nach einem Tag, nach einer Woche und nach drei Wochen aus dem Polymer ausgetreten war. Die Resultate (MMPPA im Vergleich zu DBU) gehen aus der folgenden Tabelle 2 hervor. Es zeigte sich, dass im Gegensatz zu DBU der neue Katalysator nicht eluiert wurde. Daraufhin wurde der Versuch mit einer grösseren Katalysatormenge wiederholt (Beispiele 11 und 12). Nach einem Tag konnte jetzt zwar MMPPA in der Lösung nachgewiesen werden, aber die Menge war wesentlich geringer als im entsprechenden Vergleichsversuch mit DBU, und sie erhöhte sich über die nächsten 3 Wochen praktisch nicht.

**Tabelle 2**

| Beispiel Nr. | Katalysator | Kat.-Menge [g] | 1 Tag [GC-Fläche] | 1 Woche [GC-Fläche] | 3 Wochen [GC-Fläche] |
|---|---|---|---|---|---|
| 9 | DBU | 0,1 | 3520 | 3580 | 7080 |
| 10 | MMPPA | 0,1 | 0 | 0 | 0 |
| 11 | DBU | 0,5 | 15800 | 21900 | 28200 |
| 12 | MMPPA | 0,5 | 580/1290* | 475/1350* | 590/1460* |

| | | | | | |
|---|---|---|---|---|---|
| * Die erste Zahl bezieht sich auf die 4-Methyl-Verbindung, die zweite auf die 2-Methyl-Verbindung | | | | | |

## Patentansprüche

1. Methyl-5-(3-methylpiperidino)pentanamine der allgemeinen Formel 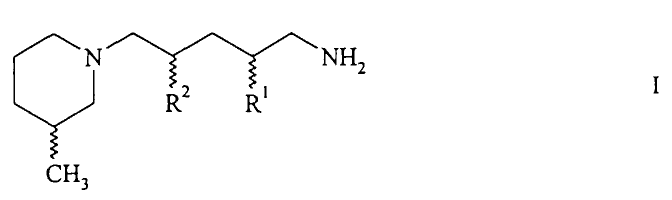 worin einer der beiden Reste R¹, R² Wasserstoff und der andere Methyl bedeutet,und deren Gemisch.

2. 2-Methyl-5-(3-methylpiperidino)pentanamin der Formel

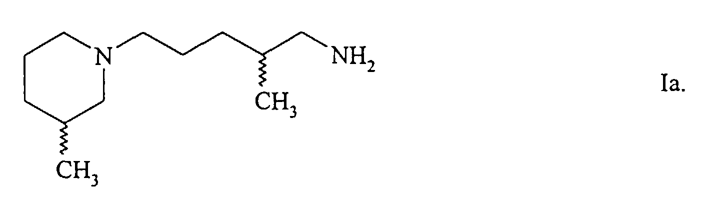

3. 4-Methyl-5-(3-methylpiperidino)pentanamin der Formel

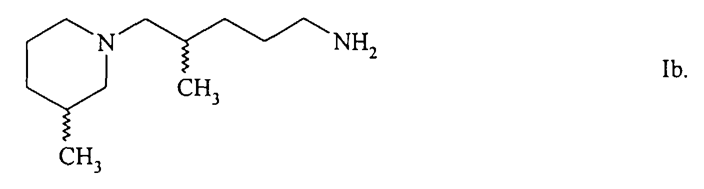

4. Verwendung der Methyl-5-(3-methylpiperidino)pentanamine gemäss Anspruch 1 als Katalysatoren zur Herstellung von Polyurethanen.
